# EUROPEAN PATENT SPECIFICATION

(11) **EP 1 575 981 B1**
(45) Date of publication and mention of the grant of the patent: **10.06.2009**
(21) Application number: 03788740.3
(22) Date of filing: 24.12.2003
(51) Int. Cl.: C07J 9/00, A61K 31/575

(54) **ANGIOGENIC ACTING TRI-SULPHATE STEROIDS AND USES THEREOF**
ANGIOGEN-WIRKENDE TRISULFAT STEROIDE UND DEREN VERWENDUNG
STEROIDES TRI-SULPHATES ANGIOGENES ET UTILISATIONS

(30) Priority: 24.12.2002 US 435864 P
(43) Date of publication of application: 21.09.2005
(73) Proprietor: THE UNIVERSITY OF BRITISH COLUMBIA, Vancouver, British Columbia, V6T 1Z3 (CA)
(72) Inventor: KARSAN, Aly, Vancouver, British Columbia V6Z 2R2 (CA); ROBERGE, Michel, Vancouver, British Columbia V6R 2H4 (CA); ANDERSEN, Raymond, Vancouver, British Columbia V6S 1Z6 (CA); POLLET, Ingrid, Anmore, British Columbia V3H 5B4 (CA)
(74) Representative: Brasnett, Adrian Hugh
(86) International application number: PCT/CA2003/002024
(87) International publication number: WO 2004/058795

(56) References cited:
- US-A- 3 836 527
- US-A- 5 336 485
- JOURNAL OF NATURAL PRODUCTS. DEC 1994, vol. 57, no. 12, December 1994 (1994-12), pages 1751-1754, XP0009027598 ISSN: 0163-3864 cited in the application
- JOURNAL OF NATURAL PRODUCTS. JAN 1994, vol. 57, no. 1, January 1994 (1994-01), pages 164-167, XP0009027599 ISSN: 0163-3864 cited in the application
- JOURNAL OF NATURAL PRODUCTS. AUG 2000, vol. 63, no. 8, August 2000 (2000-08), pages 1175-1177, XP002273368 ISSN: 0163-3864 cited in the application

## Description

The invention is in the field of angiogenesis. More specifically, the invention is in the field of compounds that promote angiogenesis.

New blood vessels develop from pre-existing vasculature in a complex physiological process known as angiogenesis. Angiogenesis involves a coordinated cascade of events initiated by the transmission of an angiogenic signal, which leads to the secretion of proteases by endothelial cells that line the inside of blood vessels, and subsequent infiltration and degradation of the basal lamina, followed by proliferation and differentiation of the endothelial cells into capillary tubes and the establishment of a new basement membrane (2). Angiogenesis is required during embryonic development for the formation of tissues and organs, and for the maintenance of organ function in the adult, for example, during endometrial cycling.

Stimulation or maintenance of appropriate angiogenesis has many therapeutic applications. For example, ischemic coronary artery disease is a major cause of morbidity and the leading cause of mortality in the west (1, 13). Current therapeutic options for patients with advanced ischemic heart disease include medical therapy or coronary revascularization by percutaneous coronary angioplasty or bypass surgery (1, 4). Many of these patients however exhibit residual symptoms of ischemia despite therapy (4). Furthermore, the incidence of restenosis or reocclusion in patients who have had invasive revascularization procedures remains high (4). The clinical situation is similar for patients with peripheral vascular disease (i.e. occlusion or stenosis of arteries other than coronary arteries) (5, 6).

Angiogenesis is also important for the prompt and appropriate healing of wounds and fractures (7), and promoting angiogenesis may hasten the healing of wounds in various situations. For instance, chronic skin ulcerations in diabetics may be treatable by improving the blood supply (8). In other situations (e.g. in burn injuries, or in chronic non-healing peptic ulcer disease) stimulating angiogenesis may promote healing (9, 10). Angiogenesis may also be important in fields that involve the growth of new tissues or organs (*in vitro* or in *vivo*), for example, for the vascularisation of synthetic skin grafts.

To date, therapeutic attempts at promoting angiogenesis have included use of Fibroblast growth factor-2 (FGF-2) and Vascular endothelial growth factor (VEGF) for treatment of myocardial and limb ischemia (11, 12). These attempts however have encountered a number of problems. First, both proteins are relatively large molecules that have to be synthesized as recombinant molecules or delivered to cells using gene therapy methods, and as such, the optimal method of delivery has not been determined, although intravenous, intra-arterial, intramuscular, and gene therapy delivery of recombinant protein have been tested (11, 12). In addition, the tissue half-life of both proteins is limited, and they can cause hypotension when delivered systemically (11,12).

With respect to small molecules, estrogen and other sex steroids have been implicated in the promotion of angiogenesis (15; 23; U.S. Patent No. 5,866,561, issued February 2, 1999 to Ungs). Estrogen however has also been reported to inhibit angiogenesis (25), and thus its role in angiogenesis may be controversial. Furthermore, administration of estrogen as a therapeutic for promoting angiogenesis may be contraindicated due to adverse effects, such as feminization in men, and the increased risk of some cancers, for example, uterine cancer or breast cancer, and blood clots in the legs in women. The ginsenoside Rg1 has been reported as having estrogen-like activity, including angiogenesis (24, 28), and beta-sitosterol, a compound derived from Aloe vera, was reported as having angiogenic activity (39,40).

Gunasekera *et al* (32) describe ophirapstanol trisulfate, a new biologically active steroid sulphate from the deep water marine sponge *Topsentia ophiraphidites.* Bifulco *et al* (33) describe HIV-inhibitory halistanol sulphate F-H. from a marine sponge, *Pseudoaxinissa digitata.* Umeyama *et al* (35) describe the compounds 24-isopropylcholesterol and 24-isopropenylcholesterol sulphate from the marine sponge *Epipolasis* species. A number of further sterol sulphate compounds are disclosed in US 3,836,527 and US 5,336,485.

### SUMMARY OF THE INVENTION

The present invention provides a compound of Formula I or pharmaceutically acceptable salt thereof, 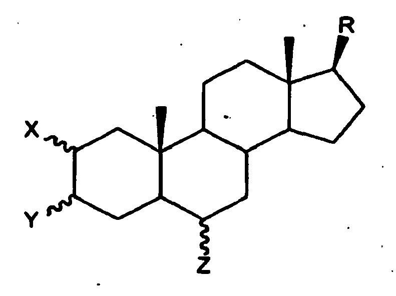 wherein
R is a linear or branched, saturated or unsaturated 1 to 15 carbon alkyl group;
wherein X, Y, and Z are all sulphate; wherein said compound is in the non-ionized form or is a pharmaceutically acceptable salt, for use in a method of treatment, wherein said method of treatment comprises administration of said compound or salt thereof to a subject for the treatment or prevention of one or more of the following: ischemia; circulatory disorders; vascular disorders; myocardial disease; pericardial disease; congenital heart disease; peripheral vascular pathologies; diabetes; coronary artery disease; atherosclerosis; infertility; insufficient endometrial vascularization; occluded blood vessels; conditions involving the pathology of endothelial cells; peptic ulcerations; endothelial ulcerations; restenosis; and wounds.

In one embodiment, the group R comprises the side chain of sokotrasterol sulphate. The compound may be sokotrasterol sulphate. The method of treatment for which the compound of formula I is used may further comprise combining said treatment with administration of vascular endothelial growth factor or fibroblast growth factor 2.

In some embodiments, the compound is substantially pure.

In some aspects, the invention may be practiced using a pharmaceutical composition including a pharmaceutically acceptable carrier and one or more compounds of formula I or pharmaceutically acceptable salts thereof as defined above.

The compound for use in the invention may be sokasterol sulphate, or may be a structure listed in Tables I or II.

The subject to be treated may be human.

Also disclosed herein is a method of identifying a sterol sulphate compound that is capable of promoting angiogenesis, the method comprising screening the compound for activity in promoting Angiogenesis. The method may include contacting the chorioallantoic membrane of a first group of chick embryos with the compound; contacting the chorioallantoic membrane of a second group of chick embryos with a composition lacking the compound; determining the angiogenic response of the first and second groups of chick embryos; and selecting a compound that increases the angiogenic response in the first group of chick embryos compared to the second group of chick embryos by at least 10%. Alternatively or additionally, the method may include contacting a first group of endothelial cells with the compound; contacting a second group of endothelial cells with a composition lacking the compound; determining the angiogenic response of the first and second groups of endothelial cells; and selecting a compound that increases the angiogenic response in the first group of endothelial cells as compared to the second group of endothelial cells by at least 10%. The endothelial cells may be human umbilical vein endothelial cells. The Angiogenic response may be determined by determining the sprouting of the endothelial cells. The compound may have the chemical structure of Formula Ia or pharmaceutically acceptable salts thereof, 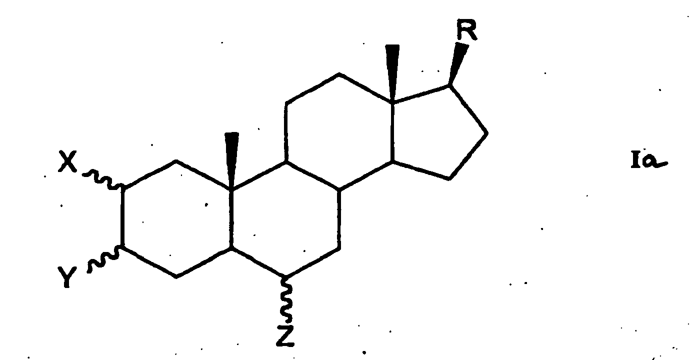 where R is a linear or branched, saturated or unsaturated one to 15 carbon alkyl group; X, Y, and Z are independently selected from H, OH, or OSO₃⁻; and at least one of X, Y, and Z is OSO₃⁻_{.}

By "promoting angiogenesis" is meant increasing or maintaining any step in the cascade of events leading to the formation of new blood vessels. These events may include, without limitation, the transmission of an angiogenic signal, migration, proliferation, differentiation, or sprouting of endothelial cells, or inhibition of apoptosis of endothelial cells. The increase or maintenance may be at least 10%, 20%, 30%, 40%, 50%, 60%, 70%, 80%, or 90%, or may be over 100%, as compared to an appropriate control.

A "disorder associated with sub-optimal angiogenesis" is any disorder that may benefit from promoting angiogenesis, as described herein or known to those of skill in the art.

### BRIEF DESCRIPTION OF THE DRAWINGS

**Figure 1** Sokotrasterol sulphate promotes endothelial sprouting *in vitro.* Dose-response curve of sokotrasterol sulphate endothelial sprouting function.
**Figure 2** Dose-response curve of endothelial sprouting function of sulphated and unsulphated compounds, including sokotrasterol sulphate, IN96-89, sokotrasterol, and cholestanol.

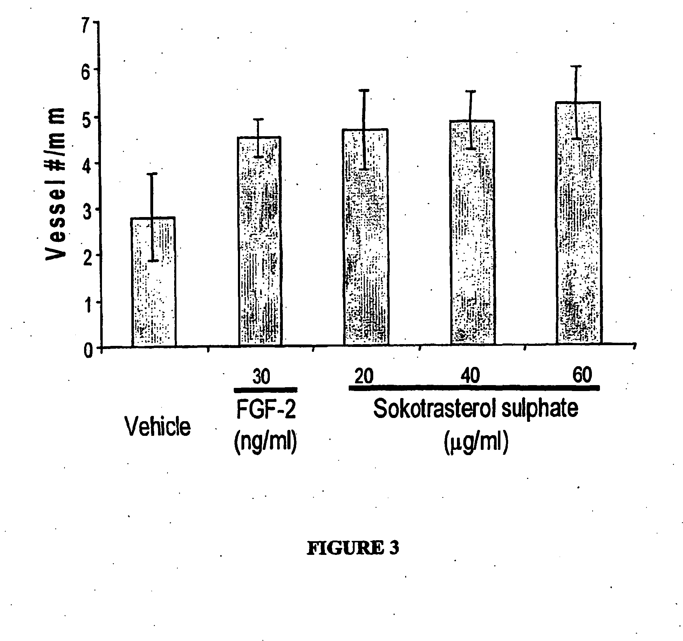

**Figure 3** Sokotrasterol sulphate promotes angiogenesis in an *in vivo* chick chorioallantoic membrane model. Quantitation by image analysis of the number of vessels entering the gelatin sponge (per mm of circumference) with different concentrations of agent.

### DETAILED DESCRIPTION OF THE INVENTION

The invention relates to sterol sulphate compounds, compositions, and methods for promoting angiogenesis, or for the preparation or identification of agents for promoting angiogenesis, in cells, tissues, cultures, organs, or organisms *in vivo* or *in vitro.* The sterol sulphate compounds and compositions are not estrogen or compounds closely related thereto. The compounds and compositions retain activity or exhibit enhanced activity upon sulphation. In some embodiments, the compounds and compositions are capable of creating new blood vessels, in contrast to the repair of an existing blood vessel (e.g., in the process of revasculatization using balloons or stents). In some embodiments, the compounds and compositions for use in the invention include small molecules that can be synthesized, and/or are unlikely to provoke an immune response, and/or have known delivery mechanisms, and/or are locally deliverable to areas of interest, and/or are minimally invasive as compared to traditional therapies such as bypass surgery or angioplasty.

### Angiogenic Compounds

Compounds for use according to the invention, in general, include all stereoisomers and enantiomers of Formula I, including those at carbons 2, 3, or 6, as identified in the conventional steroidal numbering system. In some embodiments, the compounds include saturated or unsaturated carbons in any of the tetracyclic steroidal ring system. Some embodiments have the same relative configuration of chiral centers as does sokotrasterol sulphate or are enantiomers thereof. Some embodiments have the same absolute configuration of sokotrasterol sulphate at chiral centers. In some embodiments, compounds having a side chain (R) of the precise structure of the side chain of cholesterol are specifically excluded.

In some embodiments, angiogenic compounds described herein may include compounds that are in a complex with a compound of Formula I, a chemical or breakdown product of a compound of Formula I, a derivative of a compound of Formula I, or a naturally occurring precursor of Formula I. Novel compounds of Formula I do not include the precise structures of previously described compounds, for example, those described in Tables I or II. Table I describes sterol sulphate compounds which have been reported as having activities including antifoulant, antimicrobial, antiviral (e.g., as HIV inhibitors), antileukemia, cryoprotectant, antitoxicity, 1.3-glucanase activities, or guanosine diphosphate/G-protein RAS exchange assay inhibition. Table II describes sterol sulphate compounds that have not been reported as having a biological activity.

**TABLE I**

| No. | Structure | Reference No. |
|---|---|---|
| 1 | 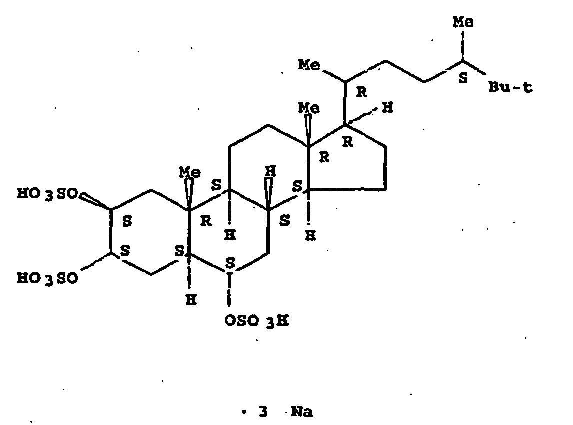 | 31 |
| 2 | 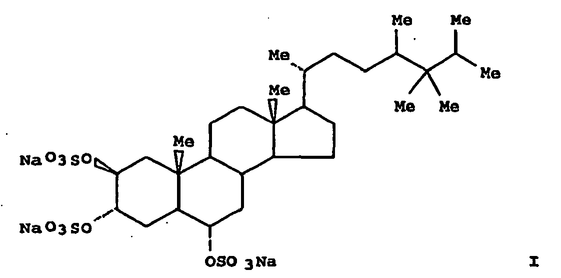 | 32 |
| 3 | 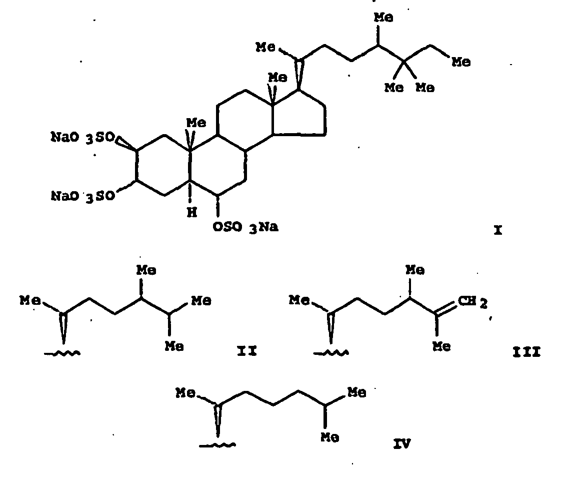 | 33 |
| | 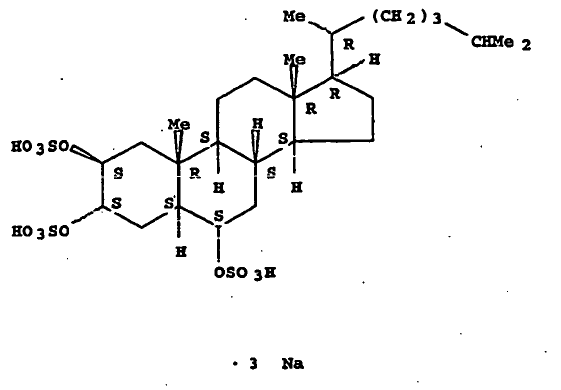 | |
| | 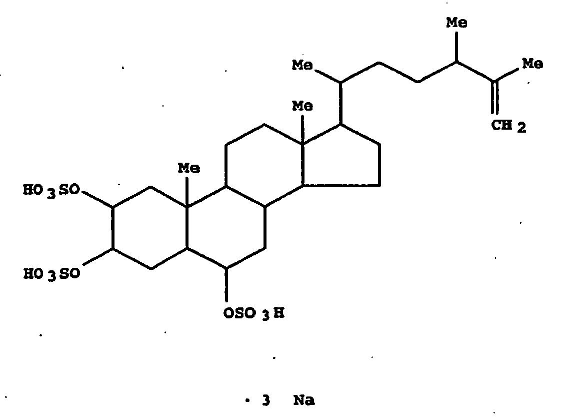 | |
| | 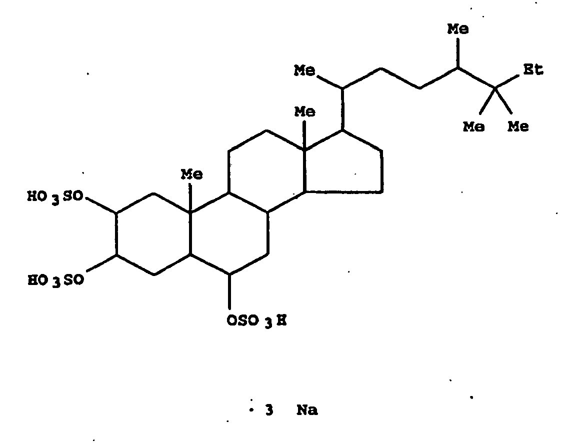 | |
| | 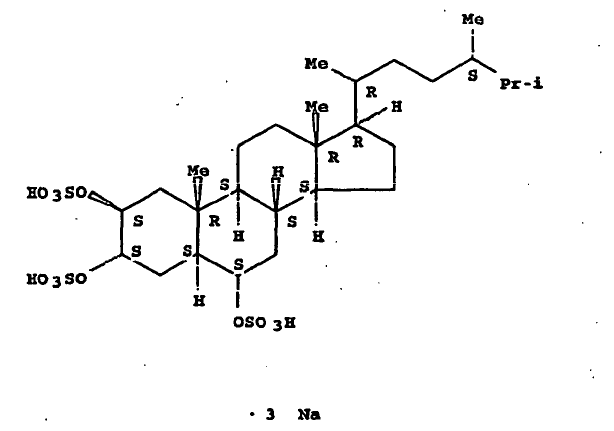 | |
| 4 | 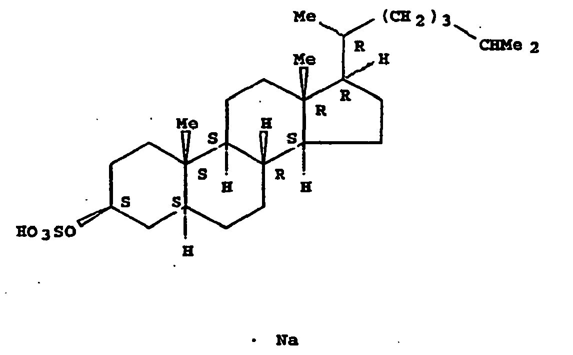 | 34 |

**TABLE II**

| No. | Structure | Reference No. |
|---|---|---|
| 1 | 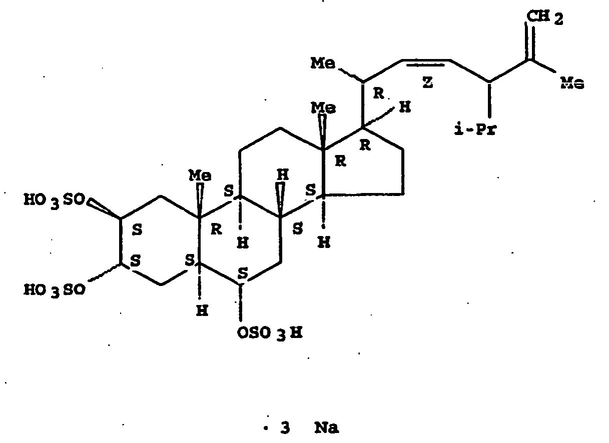 | 35 |
| 2 | 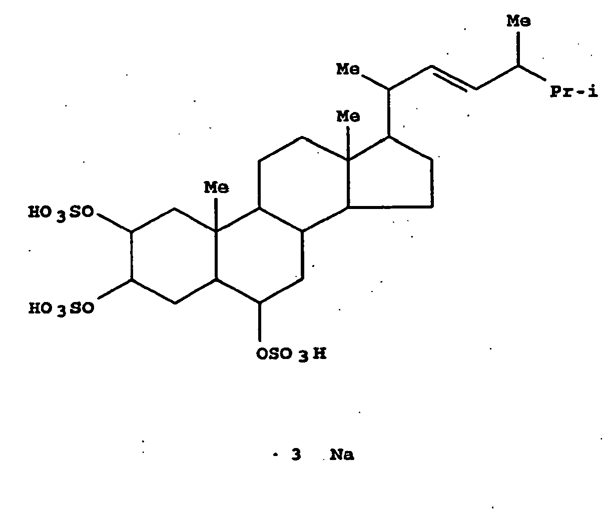 | 36 |
| | 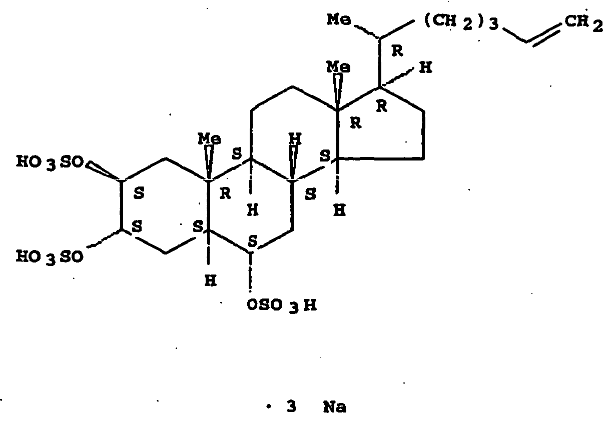 | |
| | 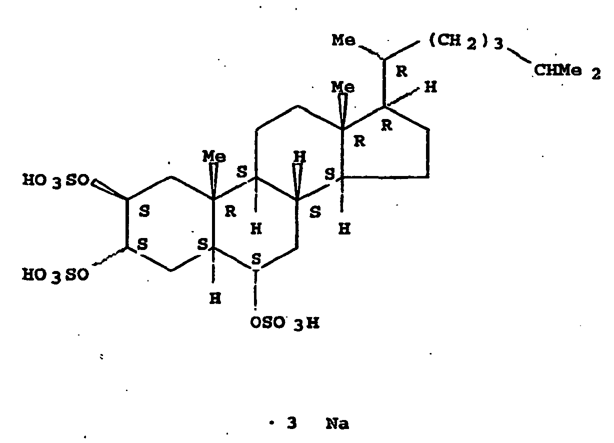 | |
| | 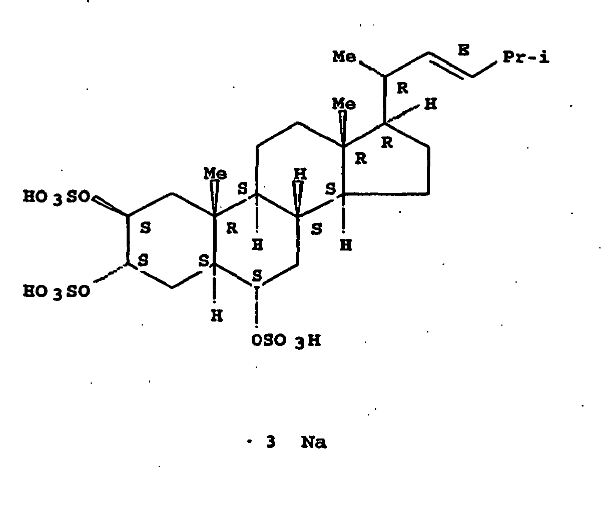 | |
| 3 | 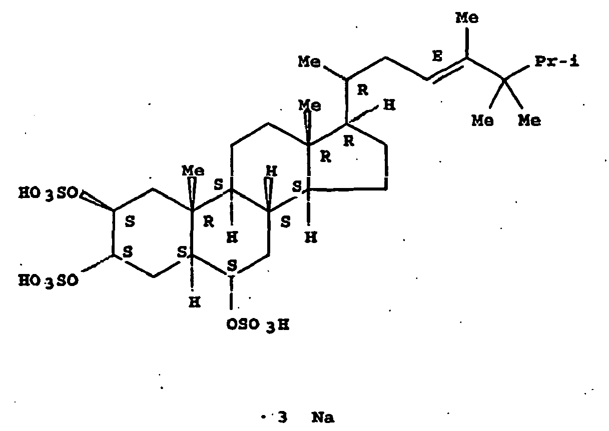 | 37 |
| 4 | 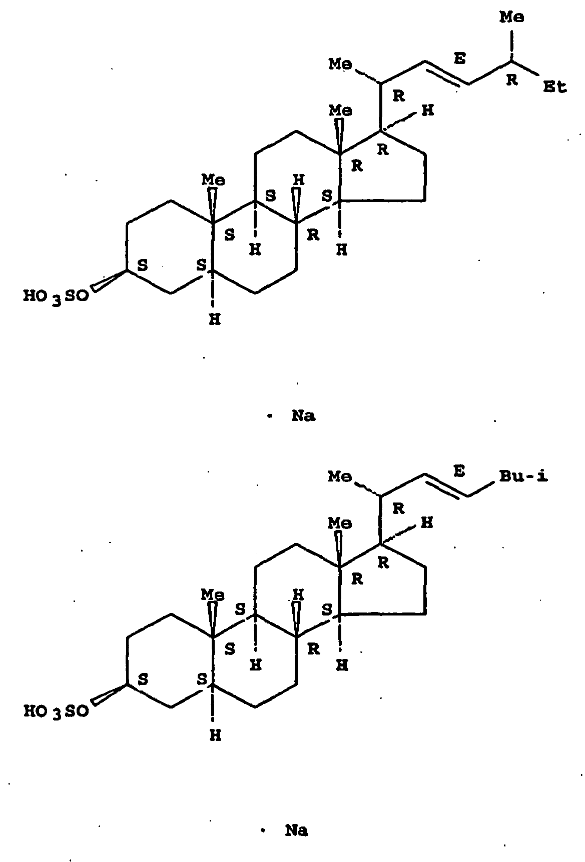 | 38 |
| | 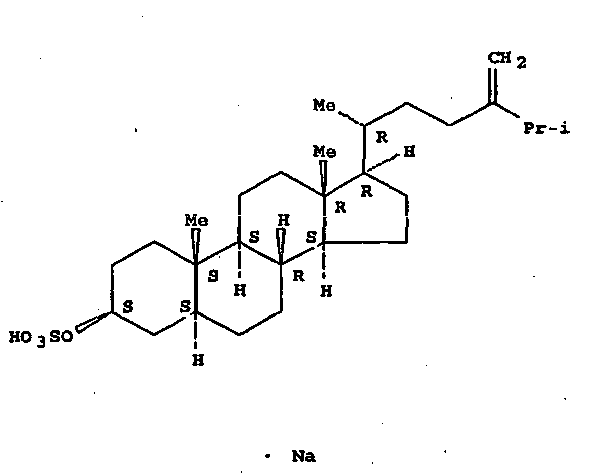 | |
| | 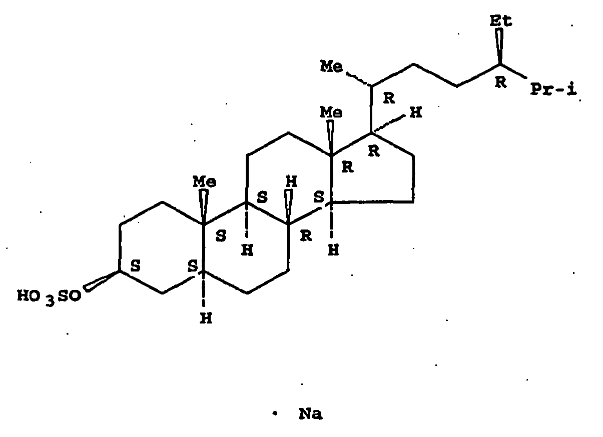 | |
| | 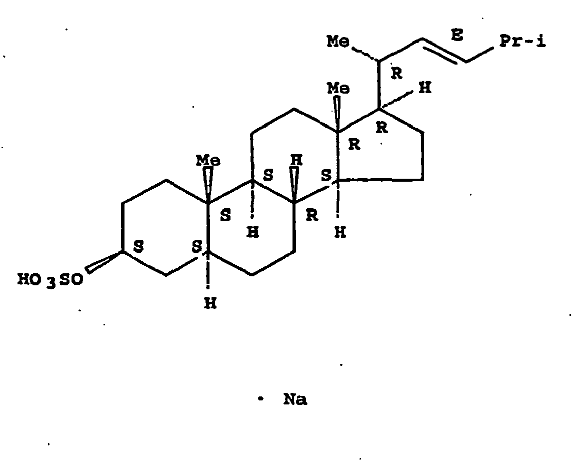 | |
| | 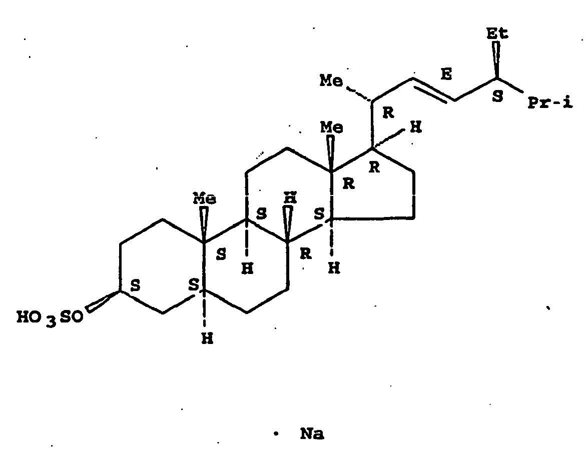 | |
| | 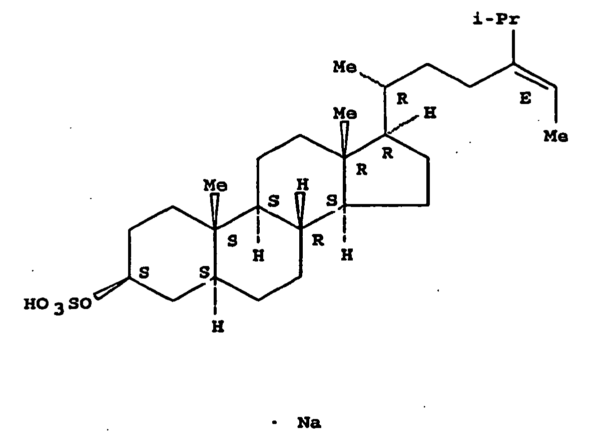 | |
| | 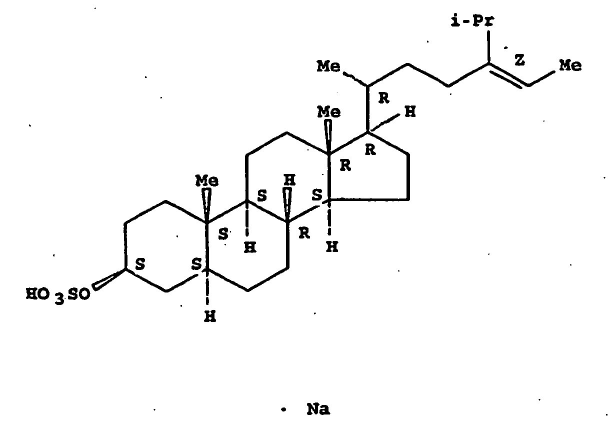 | |

Compounds and salts thereof for use in this invention are generally provided in substantially purified form. A compound or salt (if naturally occurring) is "substantially pure" or "isolated" when it is separated from the components that naturally accompany it (e.g, cells of a source organism or tissue). A compound may be substantially pure or isolated when it is substantially free of cellular contaminants, i.e, that it is present *ex vivo* and in a concentration greater than that of the compound in a source organism, tissue, or other natural source. Typically, a compound is substantially pure or isolated when it is at least 10%, 20%, 30%, 40%, 50%, or 60%, more generally 70%, 75%, 80%, or 85%, or over 90%, 95%, or 99% by weight, of the total material in a sample. Thus, for example, a compound that is chemically synthesized will be generally be substantially free from its naturally associated components. A substantially pure compound can be obtained, for example, by extraction from a natural source or by chemical syntheses. Purity can be measured using any appropriate method such as column, gas, or liquid chromatography or mass spectrometry.

### Sources and Synthesis of Compounds

Compounds or for use according to the invention, including pharmaceutically acceptable salts thereof, may be obtained by synthesis making use of common procedures as exemplified herein. Some compounds that may be used according to the invention can be obtained from natural sources. For example, compounds according to Formula I may be prepared in part or in whole from natural sources, e.g., by fractionating biological extracts (e.g., from marine organisms such as sponges or starfish, or from plants) or by derivatizing compounds available from such sources. In some embodiments, compounds according to Formula I may be prepared by total synthesis.

The synthesis schemes shown in Table **III** outline example syntheses for the sterol sulphate compounds of the invention and analogs thereof, where R is the correct steroid side chain, and P and P₁ are alcohol protecting groups (adapted from 26 and 27). For example, for compounds where R is the side chain of cholesterol, the starting material in Scheme 1 is cholesterol and in Scheme 2 is cholestanol. In some embodiments, R is a known steroid side chain. The example syntheses may be adapted to make any combination of the claimed sterol sulphates. In some embodiments, compounds may be made by partial de-sulphation of sulphated sterols as known in the art.

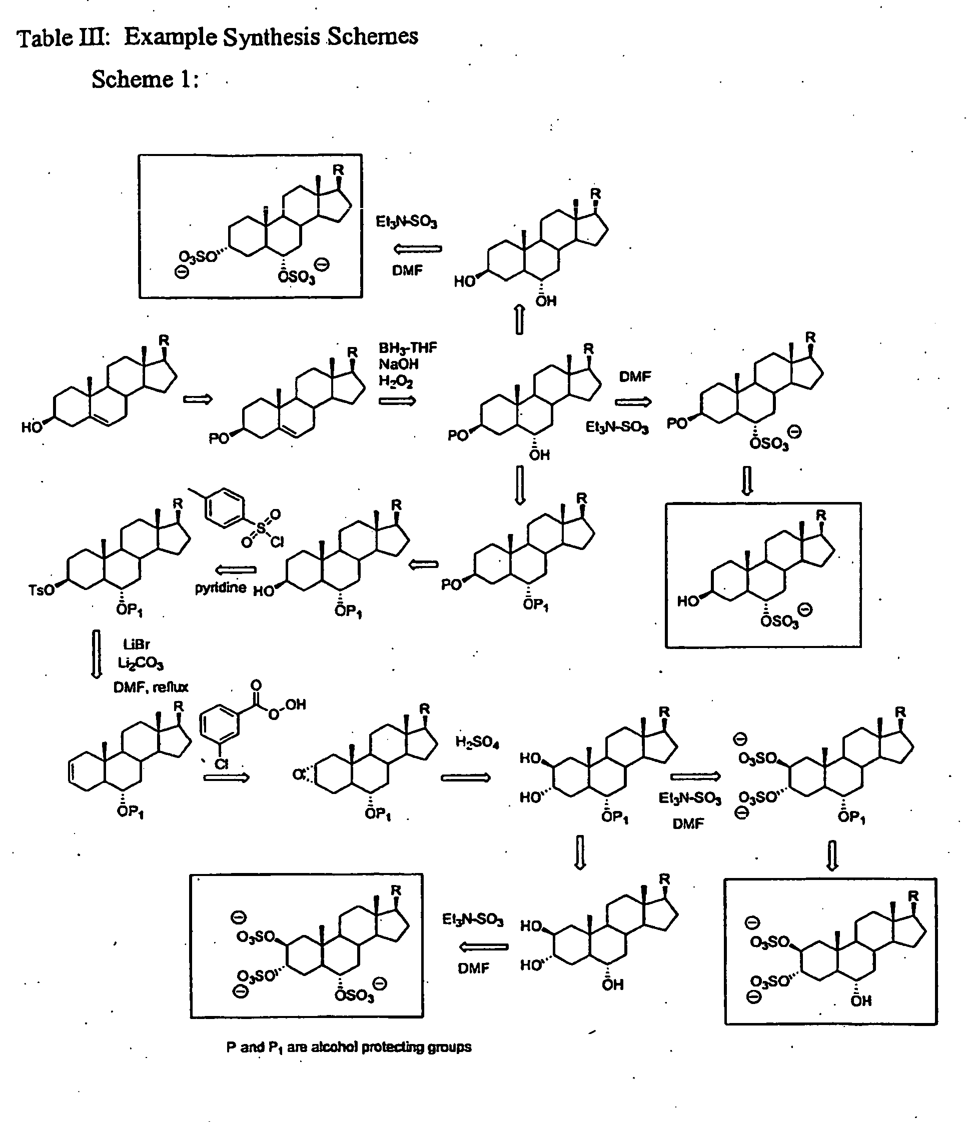

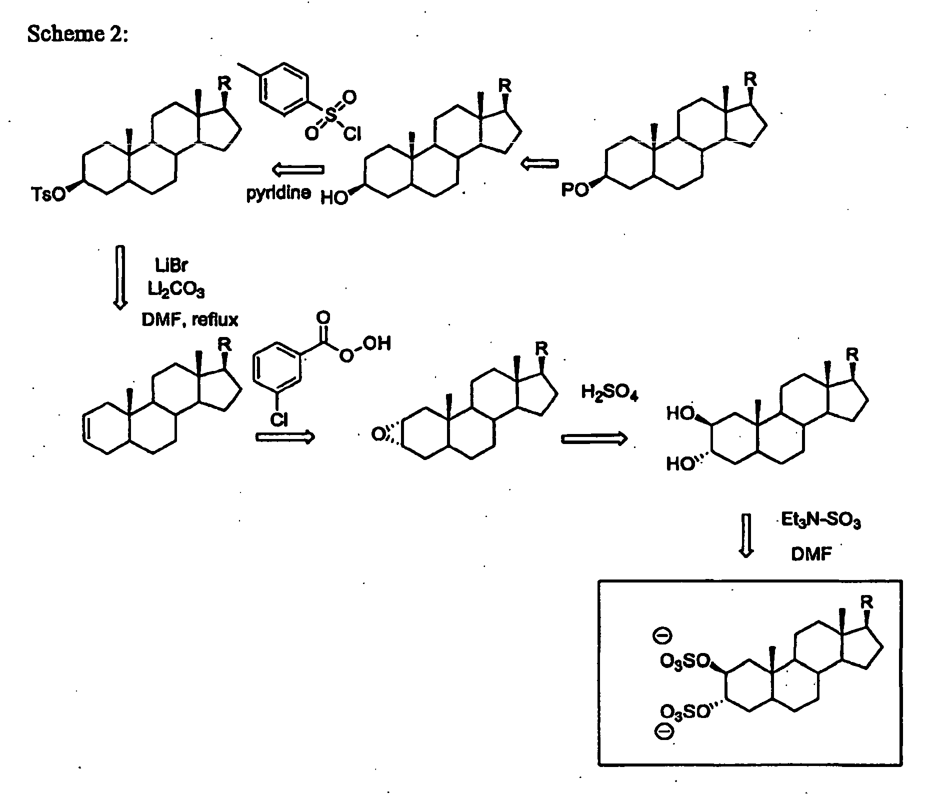

### Angiogenesis Assays

The angiogenicity of the sterol sulphates for use in the invention may be assayed using a variety of techniques, including those described herein or known to those of ordinary skill in the art, for example, attachment assays, wounding migration assays, Boyden Chamber migration assays, proliferation assays, Transwell assays, apoptosis assays, or endothelial sprouting assays (14-22). Such assays may also be used to assess the angiogenicity of compounds prepared by total synthesis as described herein, or of compounds extracted from natural sources. The angiogenicity of a compound may be determined in a number of ways, for example, relative to a control sample lacking the compound, or relative to a known angiogenic factor such as VEGF, FGF-2, angiogenin, epidermal growth factor, etc.

Angiogenesis may be assayed using suitable cells such as endothelial cells, for example, human umbilical vein endothelial cells (HUVECs). Cells and cell lines may be obtained from commercial sources, for example, ATCC, Manassas, VA, USA. Angiogenesis may also be assayed *in vivo* using suitable animal models, such as chick chorioallantoic membrane (CAM) assays, ovariectomized mice, mouse models of hindlimb ischemia, etc. (15, 29, 30). Some animal models may be obtained from, for example, The Jackson Laboratory, Bar Harbor, ME, USA.

### Disorders

The compounds, compositions, or methods of this invention may be used for the treatment or prevention of the disorders recited in claim 1.

Also disclosed herein are compounds, compositions, or methods to promote angiogenesis in for example tissues such as fibrous, muscle, endothelial, epithelial, vesicular, cardiac, cerebrovascular, vascular tissues, or avascular tissues, including the transparent structures of the eye (e.g. cornea, lens, vitreous), discs, ligaments, cartilage, tendons, epidermis etc.; organs, for example, organs for transplantation or artificial organs (e.g., heart, liver, lung, kidney, skin, pancreas, eye), or organs in need of regeneration. For tissue or organ transplants, the compounds, compositions, or methods may be applied to the tissues or organs prior to transplantation (e.g., in vitro) or may be administered to the transplant recipient (e.g., in vivo). The compounds, compositions, or methods may be used to promote angiogenesis in when using artificial implants, for example, mammary implants, penile implants, or artificial urinary sphincters, or using prostheses, to facilitate better vascularization and tolerance of the implant or prosthesis, or to inhibit restenosis of stents.

### Pharmaceutical Compositions. Administration, and Dosages

Compounds for use in the invention, for example, sokotrasterol sulphate or compounds of Formula I, may be water soluble and may be formed as salts. In general, the sulphation provides greater solubility in polar solutions, such as water or physiological solutions. In such cases, pharmaceutical compositions in accordance with this invention may include a salt of such a compound, preferably a pharmaceutically acceptable salt such as the HCl salt Other suitable salts are known in the art. The term "pharmaceutically acceptable salt" includes salts of compounds of Formula I derived from the combination of a compound of this invention and an organic or inorganic acid or base. The compounds of Formula I are useful in both non-ionized and salt form. In practice, the use of a salt form amounts to use of a base form; both forms are within the scope of the invention.

Compounds for use according to the invention can be provided alone or in combination with other compounds (for example, nucleic acid molecules, small molecules, amino acid molecules or analogs thereof), in the presence of a liposome, an adjuvant, or any pharmaceutically acceptable carrier, in a form suitable for administration to mammals, for example, humans, cattle, sheep, etc. If desired, treatment with a compound for use according to the invention may be combined with existing modes for promoting angiogenesis, such administration of VEGF or FGF-2, or with a compound that is effective in the treatment of an associated disorder.

Conventional pharmaceutical practice may be employed to provide suitable formulations or compositions, taking into account the advantages of sulphation of the compounds of the invention, to administer the compounds to subjects suffering from or presymptomatic for conditions which would benefit from the promotion of angiogenesis. Any appropriate route of administration may be employed, for example, parenteral, intravenous, subcutaneous, intramuscular, intracranial, intraorbital, ophthalmic, intraventricular, intracapsular, intraperitoneal, intranasal, aerosol, oral, topical, lavage, injection, or any other mode suitable for the selected treatment or prophylaxis. In some embodiments, the mode of administration is non-systemic, e.g., topical or local, e.g, by injection or some other means of targeted delivery to the desired organ, tissue, or cell. Compounds or pharmaceutical compositions for use in this invention, may be administered by means of a medical device or appliance such as an implant, graft, prosthesis, stent, etc. For example, a stent may be coated with such a composition for promotion of angiogenesis or the inhibition of restenosis. Also, implants may be devised that are intended to contain and release such compounds or compositions. An example would be an implant made of a polymeric material adapted to release the compound over a period of time.

Pharmaceutical compositions will typically include one or more pharmaceutically acceptable carriers or excipients suitable for the mode of administration of the preparation. As used herein "pharmaceutically acceptable carrier" or "excipient" includes any and all solvents, dispersion media, coatings, antibacterial and antifungal agents, isotonic and absorption delaying agents, and the like that are physiologically compatible. In one embodiment, the carrier is suitable for parenteral administration. Alternatively, the carrier can be suitable for intravenous, intraperitoneal, intramuscular, sublingual or oral administration. Pharmaceutically acceptable carriers include sterile aqueous solutions or dispersions and sterile powders for the extemporaneous preparation of sterile injectable solutions or dispersion. The use of such media and agents for pharmaceutically active substances is well known in the art. Except insofar as any conventional media or agent is incompatible with the active compound, use thereof in the pharmaceutical compositions of the invention is contemplated. Supplementary active compounds can also be incorporated into the compositions. Suitable carriers are those known in the art for use in the selected modes of administration.

Methods well known in the art for making formulations are found in, for example, "Remington's Pharmaceutical Sciences" (19th edition), ed. A. Gennaro, 1995, Mack Publishing Company, Easton, Pa. Formulations for parenteral administration may, for example, contain excipients, sterile water, or saline, polyalkylene glycols such as polyethylene glycol, oils of vegetable origin, or hydrogenated napthalenes. Biocompatible, biodegradable lactide polymer, lactide/glycolide copolymer, or polyoxyethylene-polyoxypropylene copolymers may be used to control the release of the compounds. Other potentially useful parenteral delivery systems for angiogenic compounds include ethylenevinyl acetate copolymer particles, osmotic pumps, implantable infusion systems, and liposomes. Formulations for inhalation may contain excipients, for example, lactose, or may be aqueous solutions containing, for example, polyoxyethylene-9-lauryl ether, glycocholate and deoxycholate, or may be oily solutions for administration in the form of nasal drops, or as a gel. Formulations may be in the form of liquid solutions or suspensions; for oral administration, formulations may be in the form of tablets or capsules.

For therapeutic or prophylactic compositions, the compounds are administered to an individual in an effective amount, i.e., an amount sufficient to promote angiogenesis, depending on the disorder. An "effective amount" of a compound according to the invention includes a therapeutically effective amount or a prophylactically effective amount. A "therapeutically effective amount" refers to an amount effective, at dosages and for periods of time necessary, to achieve the desired therapeutic result, such as promotion of angiogenesis. A therapeutically effective amount of a compound may vary according to factors such as the disease state, age, sex, and weight of the individual, and the ability of the compound to elicit a desired response in the individual. Dosage regimens may be adjusted to provide the optimum therapeutic response. A therapeutically effective amount is also one in which any toxic or detrimental effects of the compound are outweighed by the therapeutically beneficial effects. A "prophylactically effective amount" refers to an amount effective, at dosages and for periods of time necessary, to achieve the desired prophylactic result, such as promotion of angiogenesis. Typically, a prophylactic dose is used in subjects prior to or at an earlier stage of disease, so that a prophylactically effective amount may be less than a therapeutically effective amount. A preferred range for therapeutically or prophylactically effective amounts of a compound may be any integer from 0.1 nM-0.1M, 0.1 nM-0.05M, 0.05 nM-15µM or 0.01 nM-10µM. Such dosages are readily determinable by persons of ordinary skill in the relevant art. An "angiogenesis promoting amount" of a compound is an amount that is sufficient to promote angiogenesis, as determined for example using an angiogenesis assay as described herein or known in the art.

It is to be noted that dosage values may vary with the severity of the condition to be alleviated. For any particular subject, specific dosage regimens may be adjusted over time according to the individual need and the professional judgement of the person administering or supervising the administration of the compositions. Dosage ranges set forth herein are exemplary only and do not limit the dosage ranges that may be selected by medical practitioners. The amount of active compound in the composition may vary according to factors such as the disease state, age, sex, and weight of the individual. Dosage regimens may be adjusted to provide the optimum therapeutic response. For example, a single bolus may be administered, several divided doses may be administered over time or the dose may be proportionally reduced or increased as indicated by the exigencies of the therapeutic situation. It may be advantageous to formulate parenteral compositions in dosage unit form for ease of administration and uniformity of dosage.

In general, compounds of the invention should be used without causing substantial toxicity. Toxicity of the compounds of the invention can be determined using standard techniques, for example, by testing in cell cultures or experimental animals and determining the therapeutic index, i.e., the ratio between the LD50 (the dose lethal to 50% of the population) and the LD100 (the dose lethal to 100% of the population). In some circumstances however, such as in severe disease conditions, it may be necessary to administer substantial excesses of the compositions. Ultimately, dosage and duration of treatment is determined by the practitioner in accordance with standard protocols and information concerning the activity and toxicity of the chosen compound.

Various alternative embodiments and examples of the invention are described herein. These embodiments and examples are illustrative and should not be construed as limiting the scope of the invention.

### EXAMPLES

### Identification of Angiogenic Compounds

A collection of crude natural extracts were screened to identify small molecules that would promote the sprouting of endothelial cells *in vitro,* as described below. Three extracts out of a total of sixty tested had the ability to induce endothelial sprouting. Purification of one of these extracts led to the identification of sokotrasterol sulphate, a sulphated steroid with an extensively methylated side chain, isolated from a marine sponge collected in the Dominica Republic, and having the following chemical structure (3):

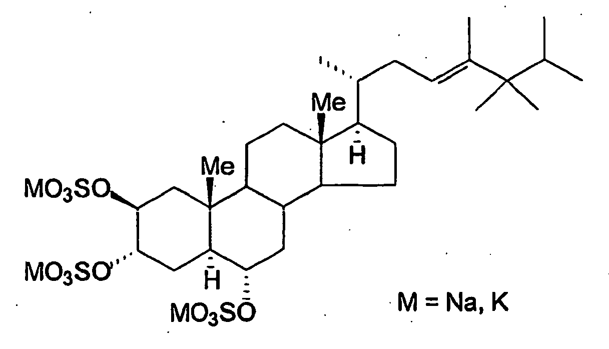

As used herein the term "sokotrasterol sulphate" refers to the trisulphated compound, and "sokotrasterol" refers to a compound that lacks sulphation, but possesses the same side chain as sokotrasterol sulphate.

### Endothelial sprouting assay

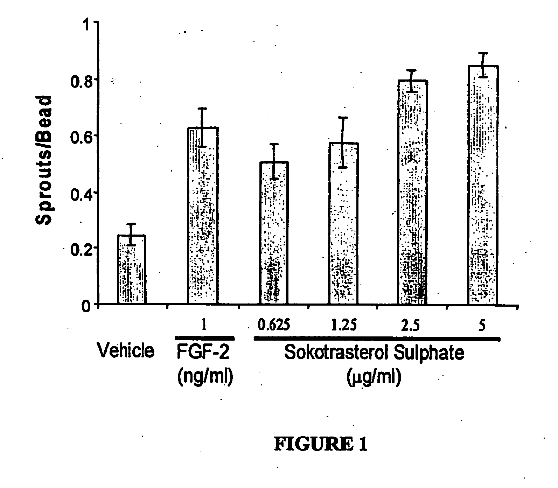

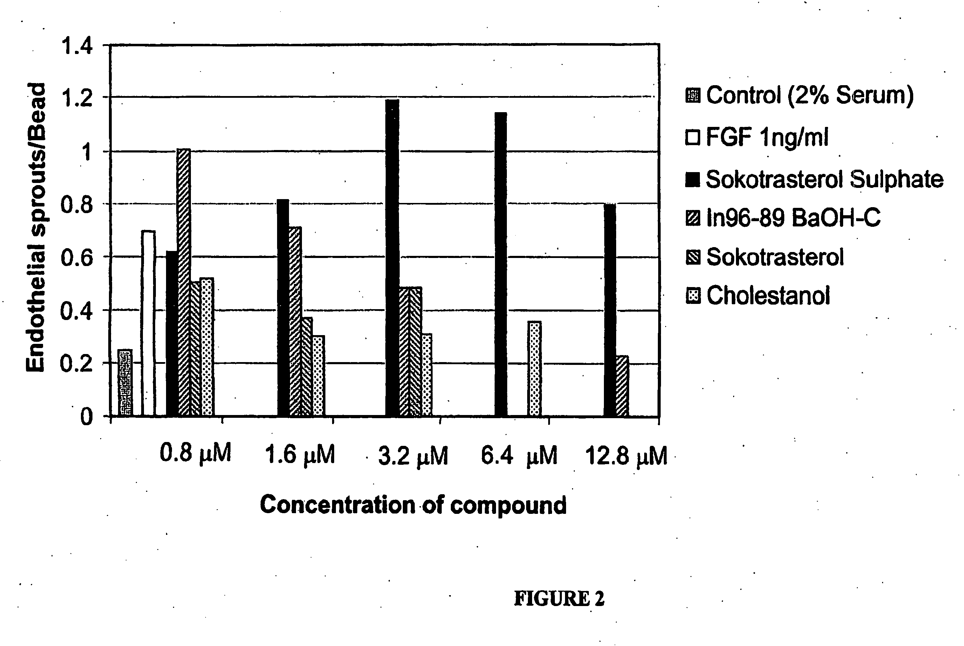

Human umbilical vein endothelial cells (HUVEC) from normal umbilical cords were obtained by collagenase treatment. Cultures were propagated in MCDB medium supplemented with 15% FCS, heparin, and endothelial cell growth supplement. Endothelial sprouting was assessed by a modification of the method of Nehls and Drenckhahn¹⁴. Briefly, microcarrier beads coated with gelatin (Cytodex 3, Sigma) were seeded with HUVEC. When the cells reached confluence on the beads, equal numbers of HMEC-coated beads were embedded in fibrin gels in 96-well plates. For preparation of fibrin gels, bovine fibrinogen was dissolved in MCDB at a concentration of 2.5 mg/ml. Aprotinin was added at a concentration of 0.05 mg/ml and the solution filtered through a 0.22 micron filter. Fibrinogen solution was supplemented with FGF-2 or sokotrasterol sulphate. As a control, fibrinogen solution without angiogenic factor was used. Following transfer of the fibrinogen solution to 96-well plates, HUVEC-coated beads were added at a density of 50 beads/well, and clotting was induced by the addition of thrombin (1.2 U/ml). After clotting was complete, gels were equilibrated with MCDB + 2% FCS at 37°C. Following 60 min of incubation, the overlying medium was changed for all wells. MCDB + 2% FCS alone or containing FGF-2 (1 ng/ml) or sokotrasterol (from 0.625 µg/ml to 5 µg/ml) was added to the wells. After 3 days of incubation with daily medium changes, the number of capillary-like tubes formed was quantitated by counting the number of tube-like structures per microcarrier bead (sprouts/bead). Only sprouts greater than 150 µm in length and composed of at least 3 endothelial cells were counted. Analysis of sokotrasterol sulphate showed that it promoted endothelial sprouting *in vitro* in a concentration-dependent manner (Figure 1). Data in Figure 1 are averages from seven independent experiments. The endothelial sprouting assay was also performed using sokotrasterol (i.e., not sulphated), cholestanol (not sulphated), and a trisulphate of cholestane (IN 98-89, having the chemical structure shown below), in addition to sokotrasterol sulphate (Figure 2). Both sulphated compounds exhibited enhanced angiogenic activity when compared with their un-sulphated analogs.

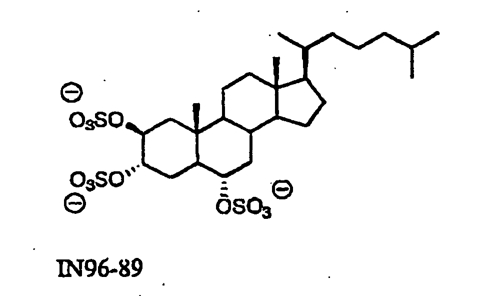

### Chick chorioallantoic membrane (CAM assay

Fertilized White Leghorn chicken eggs (*Gallus gallus domesticus*) were incubated at 37°C under conditions of constant humidity. All chick eggs were handled according to institutional animal care procedures. On embryonic day 6, the developing chick chorioallantoic membrane (CAM) was separated from the shell by opening a small circular window at the broad end of the egg above the air sac. The embryos were checked for normal development, the window sealed with Parafilm, and the eggs returned to the incubator for 2 more days. On day 8, CAMs were treated with FGF-2 (30 ng/ml), sokotrasterol sulphate (20, 40 and 60 µg/ml) or PBS by loading 20 µl onto 2-mm³ gelatin sponges (Gelfoam; Pharmacia Upjohn), which were then placed on the surface of the developing CAM. Eggs were resealed and returned to the incubator. On day 10, images of the CAMs were captured digitally using an Olympus SZX9 stereomicroscope (Olympus America) equipped with a Spot RT digital imaging system (Diagnostic Instruments). Neovascularization was quantitated for each CAM by counting the number of vessels that entered the sponge area, and dividing by the perimeter of the sponge (vessels/mm). Northern Eclipse version 6.0 (Empix Imaging Inc.) was used for manual vessel counting and mesh perimeter measurements. Thus, there was a positive concentration-dependent effect of sokotrasterol sulphate in the promotion of new blood vessel growth in an *in vivo* model of angiogenesis on the chick chorioallantoic membrane (Figure 2).

No toxicity was noted at any of the test concentrations in either the endothelial sprouting or the CAM assay.

### REFERENCES

1. Helisch A, Ware JA. Therapeutic angiogenesis for ischemic heart disease. Adv Exp Med Biol. 2000;476:327-350.
2. Karsan A, Harlan JM: The blood vessel wall, in Hoffman R, Benz EJJ, Shattil SJ, Furie B, Cohen HJ, Silberstein LE, McGlave P (eds): Hematology: Basic Principles and Practice. New York, Churchill Livingstone, 1999, p 1770-1782
3. Makarieva TN, Shubina LK, Kalinovsky AI, Stonik VA, Elyakov GB. Steroids in Porifera II. Steroid derivatives from two sponges of the family Halichondriidae. Sokotrasterol sulphate, a marine steroid with a new pattern of side chain alkylation. Steroids. 1983;42:267-281.
4. Solomon AJ, Gersh BJ. Management of chronic stable angina: medical therapy, percutaneous transluminal coronary angioplasty, and coronary artery bypass graft surgery. Lessons from the randomized trials. Ann Intern Med. 1998;128:216-223.
5. Criqui MH. Peripheral arterial disease--epidemiological aspects. Vasc Med. 2001;6:3-7.
6. Dieter RS, Chu WW, Pacanowski JP, Jr., McBride PE, Tanke TE. The significance of lower extremity peripheral arterial disease. Clin Cardiol. 2002;25:3-10.
7. Tonnesen MG, Feng X, Clark RA. Angiogenesis in wound healing. J Investig Dermatol Symp Proc. 2000;5:40-46.
8. Beckman JA, Creager MA, Libby P. Diabetes and atherosclerosis: epidemiology, pathophysiology, and management. Jama. 2002;287:2570-2581.
9. Gibran NS, Heimbach DM. Current status of burn wound pathophysiology. Clin Plast Surg. 2000;27:11-22.
10. Tarnawski A, Szabo IL, Husain SS, Soreghan B. Regeneration of gastric mucosa during ulcer healing is triggered by growth factors and signal transduction pathways. J Physiol Paris. 2001;95:337-344.
11. Post MJ, Laham R, Sellke FW, Simons M. Therapeutic angiogenesis in cardiology using protein formulations. Cardiovasc Res. 2001;49:522-531.
12. Hammond HK, McKinan MD. Angiogenic gene therapy for heart disease: a review of animal studies and clinical trials. Cardiovasc Res. 2001;49:561-567.
13. Heart Disease and Stroke in Canada, Economic Impact of Cardiovascular Disease. Health Canada, Health Protection Branch - Laboratory Centre for Disease Control. 1997.
14. Nehls, V. and Drenckhahn, D. A novel, microcarrier-based in vitro assay for rapid and reliable quantification of three-dimensional cell migration and angiogenesis. Microvasc. Res. 1995;50: 311-22.
15. Morales DE, McGowan KA, Grant DS, Maheshwari S, Bhartiya D, Cid MC, Kleinman HK, Schnaper HW. Estrogen promotes angiogenic activity in human umbilical vein endothelial cells in vitro and in a murine model. Circulation. 1995 Feb 1;91(3):755-63.
16. Leong KG, Hu X, Li L, Noseda M, Larrivee B, Hull C, Hood L, Wong F, Karsan A. Activated Notch4 inhibits angiogenesis: role of beta 1-integrin activation. Mol Cell Biol. 2002;22:2830-2841.
17. Karsan A, Yee E, Harlan JM. Endothelial cell death induced by tumor necrosis factor-alpha is inhibited by the Bcl-2 family member, A1. J Biol Chem. 1996;271:27201-27204.
18. Karsan A, Yee E, Poirier GG, Zhou P, Craig R, Harlan JM. Fibroblast growth factor-2 inhibits endothelial cell apoptosis by Bcl-2-dependent and independent mechanisms. Am J Pathol. 1997;151:1775-1784.
19. Duriez PJ, Wong F, Dorovini-Zis K, Shahidi R, Karsan A. A1 functions at the mitochondria to delay endothelial apoptosis in response to tumor neurosis factor. J Biol Chem. 2000;275:18099-18107.
20. Hu X, Yee E, Harlan JM, Wong F, Karsan A. Lipopolysaccharide induces the antiapoptotic molecules, A1 and A20, in microvascular endothelial cells. Blood. 1998;92:2759- 2765.
21. Hull C, McLean G, Wong F, Duriez PJ, Karsan A. Lipopolysaccharide signals an endothelial apoptosis pathway through TNF receptor-associated factor 6-mediated activation of c-Jun NH2-terminal kinase. J Immunol. 2002;169:2611-2618.
22. Bombeli T, Karsan A, Tait JF, Harlan JM. Apoptotic vascular endothelial cells become procoagulant. Blood. 1997;89:2429-2442.
23. Losordo DW, Isner JM. Estrogen and angiogenesis: A review. Arterioscler Thromb Vasc Biol. 2001 Jan;21(1):6-12.
24. Chan RY, Chen WF, Dong A, Guo D, Wong MS, Estrogen-like activity of ginsenoside Rg1 derived from Panax notoginseng. J Clin Endocrinol Metab. 2002 Aug;87(8):3691-5.
25. Ma W, Tan J, Matsumoto H, Robert B, Abrahamson DR, Das SK, Dey SK, Adult tissue angiogenesis: evidence for negative regulation by estrogen in the uterus. Mol Endocrinol. 2001 Nov;15(11):1983-92.
26. G.A. Garrido santos, A.P. Murray, C.A. Pujol, E.B. Damonte and M.S. Maier. Steroids, 2003, 68, 125-132.
27. M.E. Jung and T.W. Johnson. Tetrahedron 2001, 57, 1449-1481.
28. Fan, Tai-Ping, D. and Stephen Hu, Differential Effects Of Ginsenosides On Endothelial Cell Biology, Abstract, Angiogenesis 2 Euroconference, Paris, France, June 19-20, 2003.
29. Couffinhal T, Silver M, Zheng LP, Kearney M, Witzenbichler B, Isner JM. Mouse model of angiogenesis. Am J Pathol. 1998;152:1667-1679.
30. Scholz D, Ziegelhoeffer T, Helisch A, Wagner S, Friedrich C, Podzuweit T, Schaper W. Contribution of arteriogenesis and angiogenesis to postocclusive hindlimb perfusionin mice. J Mol Cell Cardiol. 2002;34:775-787.
31. Tsukamoto, Sachiko; Kato, Haruko; Hirota, Hiroshi; Fusetani, Nobuhiro. Fusetani Biofouling Project, Research Development Corporation Japan, Niigata Engineering Company Ltd., Yokohama, Japan. Biofouling (1997), 11(4), 283-291.
32. Gunasekera, Sarath P.; Sennett, Susan H.; Kelly-Borges, Michelle; Bryant, Robert W. Harbor Branch Oceanographic Institution, Inc., Fort Pierce, FL, USA. Journal of Natural Products (1994), 57(12), 1751-4.
33. Bifulco, G.; Bruno, I.; Minale, L.; Riccio, R. Dip. Chim. Sostanze Natl., Univ. Napoli "Federico II", Naples, Italy. Journal ofNatural Products (1994), 57(1), 164-7.
34. Fariss, Marc W. (Virginia Commonwealth University, USA). U.S. (1997), 54 pp., Cont.-in-part of U.S. Ser. 5,336,485.
35. Umeyama, Akemi; Adachi, Kanako; Ito, Seiichi; Arihara, Shigenobu. Faculty of Pharmaceutical Sciences, Tokushima Bunri University, Tokushima, Japan. Journal of Natural Products (2000), 63(8), 1175-1177.
36. Kanazawa, Satoshi; Fusetani, Nobuhiro; Matsunaga, Shigeki. Fac. Agric., Univ. Tokyo, Tokyo, Japan. Tetrahedron (1992), 48(26), 5467-72.
37. Il'in, S. G.; Reshetnyak, M. V.; Shchedrin, A. P.; Makarieva, T. N.; Shubina, L. K.; Stonik, V. A.; Elyakov, G. B.; Sobolev, A. N. Pac. Inst. Bioorg. Chem., Vladivostok, USSR. Journal of Natural Products (1992), 55(2), 232-6.
38. Makarieva, Tatyana N.; Stonik, Valentin A.; Kapustina, Irina 1; Boguslavsky, Valentin M.; Dmitrenoik, Andrei S.; Kalinin, Vladimir I.; Cordeiro, M. Lucinda; Djerassi, Carl. Lab. Chem. Mar. Nat. Prod., Pac. Inst. Bioorg. Chem., Vladivostok, Russia. Steroids (1993), 58(11), 508-17.
39. Moon EJ, Lee YM, Lee OH, Lee MJ, Lee SK, Chung MH, Park YI, Sung CK, Choi JS, Kim KW, A novel angiogenic factor derived from Aloe vera gel: beta-sitosterol, a plant sterol. Angiogenesis. 1999;3(2):117-23.
40. Choi S, Kim KW, Choi JS, Han ST, Park YI, Lee SK, Kim JS, Chung MH. Angiogenic activity of beta-sitosterol in the ischaemia/reperfusion-damaged brain of Mongolian gerbil. Planta Med. 2002 Apr;68(4):330-5.

## Claims

1. A compound of Formula I or pharmaceutically acceptable salt thereof, 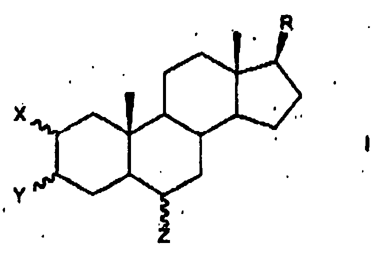 wherein
R is a linear or branched, saturated or unsaturated 1 to 15 carbon alkyl group;
wherein X, Y, and 2 are all sulphate: wherein said compound is in the non-ionized form or is a pharmaceutical acceptable salt, for use in a method of treatment or prevention of one or more of the following: ischemia; circulatory disorders; vascular disorders; myocardial disease; pericardial disease; congenital heart disease; peripheral vascular pathologies; diabetes; coronary artery disease; atherosclerosis; infertility; insufficient endometrial vascularization, occluded blood vessels, conditions involving the pathology of endothelial calls; peptic ulcerations; endothelial ulcerations; restenosis; and wounds.

2. The compound for use according to claim 1, wherein R comprises the side chain of sokotresterol sulphate.

3. The compound for use according to claim 1, wherein said compound is sokotrasterol sulphate.

4. The compound for use according to claim 1, wherein said method further comprises combining said treatment with administration of vascular endothelial growth factor or fibroblast growth factor 2.

5. The compound for use according to any of the preceding claims, wherein the ischemia is selected from the group consisting of ischemic stroke, cerebral ischemia, myocardial ischemia, intestinal ischemia, retinal or ocular ischemia, and spinal ischemia.

## Patentansprüche

1. Verbindung der Formel I oder ein pharmazeutisch annehmbares Salz davon, 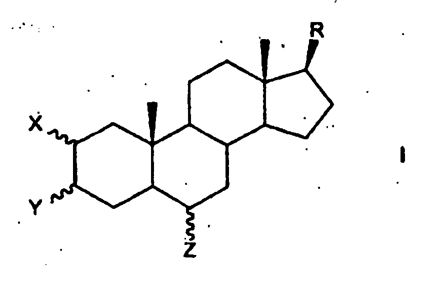 worin
R eine unverzweigte oder verzweigte, gesättigte oder ungesättigte Alkylgruppe mit 1 bis 15 Kohlenstoffen ist;
worin X, Y und Z alle Sulfate sind;
worin die Verbindung in nicht ionisierter Form vorliegt oder ein pharmazeutisch annehmbares Salz ist, zur Verwendung in einem Verfahren zur Behandlung oder Prävention einer oder mehrerer der folgenden Erkrankungen: Ischämie; Kreislaufstörungen; Gefäßstörungen; Myokarderkrankungen; Perikarderkrankungen; angeborenen Herzerkrankungen; peripheren Gefäßerkrankungen; Diabetes; Koronararterien-Krankheiten; Atherosklerose; Unfruchtbarkeit; unzureichender Gebärmutterschleimhautgefäßbildung; Blutgefäßverschlüssen; die Pathologie von Endothelzellen betreffenden Erkrankungen; Ulcus pepticum; Endothelulzerationen; Restenose; und Wunden.

2. Verbindung zur Verwendung nach Anspruch 1, worin R die Seitenkette von Sokotrasterolsulfat umfasst.

3. Verbindung zur Verwendung nach Anspruch 1, worin die Verbindung Sokotrasterolsulfat ist.

4. Verbindung zur Verwendung nach Anspruch 1, worin das Verfahren weiters das Kombinieren der Behandlung mit der Verabreichung von Gefäßendothelwachstumsfaktor oder Fibroblastenwachstumsfaktor 2 umfasst.

5. Verbindung zur Verwendung nach einem der vorangegangenen Ansprüche, worin Ischämie aus der aus einem ischämischen Schlaganfall, einer Hirnischämie, einer Myokardischämie, einer Darmischämie, einer retinalen Ischämie oder einer o-kularen Ischämie und einer Rückenmarksischämie bestehenden Gruppe ausgewählt ist.

## Revendications

1. Composé de la Formule I ou un sel pharmaceutiquement acceptable de celui-ci, 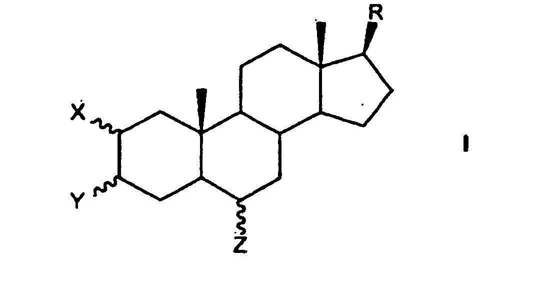 où
R est un groupe alkyle linéaire ou branché, saturé ou non saturé de 1 à 15 carbones;
où X, Y et Z sont tous sulfates;
où ledit composé se présente sous la forme non-ionisée
ou est un sel pharmaceutiquement acceptable, pour utilisation dans une méthode de traitement ou de prévention d'un ou de plusieurs des suivants: ischémie; troubles de la circulation; troubles vasculaires; affection du myocarde; affection péricardique; maladie de coeur congénitale; pathologies vasculaires périphériques; diabètes; maladie des artères coronaires; athérosclérose; stérilité; vascularisation endométriale insuffisante; occlusion de vaisseaux sanguins; conditions impliquant la pathologie de cellules endothéliales; ulcérations peptiques; ulcérations endothéliales; resténose; et plaies.

2. Composé pour utilisation selon la revendication 1, où R comprend la chaîne latérale de sokotrastérol sulfate.

3. Composé pour utilisation selon la revendication 1, où ledit composé est du sokotrastérol sulfate.

4. Composé pour utilisation selon la revendication 1, où ladite méthode comprend en outre la combinaison dudit traitement avec l'administration d'un facteur de croissance endothéliale vasculaire ou facteur de croissance de fibroblaste 2.

5. Composé pour utilisation selon l'une quelconque des revendications précédentes, où l'ischémie est sélectionnée dans le groupe consistant en accident ischémique cérébral, ischémie cérébrale, ischémie du myocarde, ischémie intestinale, ischémie rétinale ou oculaire et ischémie spinale.
